# EUROPÄISCHE PATENTSCHRIFT

(11) **EP 2 238 921 B1**
(45) Veröffentlichungstag und Bekanntmachung des Hinweises auf die Patenterteilung: **29.06.2011**
(21) Anmeldenummer: 10003646.6
(22) Anmeldetag: 01.04.2010
(51) Int. Cl.: A61B 17/225, A61H 23/00

(54) **Vorrichtung zur Behandlung des menschlichen oder tierischen Körpers durch mechanische Druckwellen mit einem austauschbaren Prallkörper**
Device for treating the human or animal body with mechanical pressure waves with a replaceable impact body
Dispositif de traitement du corps d'un homme ou d'un animal par ondes de pression mécaniques à l'aide d'un corps d'impact échangeable

(30) Priorität: 03.04.2009 DE 202009004616 U
(43) Veröffentlichungstag der Anmeldung: 13.10.2010
(73) Patentinhaber: STORZ MEDICAL AG, 8274 Tägerwilen (CH)
(72) Erfinder: Novak, Pavel, Dr., 8234 Stetten, SH (CH); Schulz, Manfred, 8274 Tägerwilen/T (CH); Froese, Klaus, 78465 Konstanz (DE)
(74) Vertreter: Szynka, Dirk

(56) Entgegenhaltungen:
- EP-A1- 1 504 724
- WO-A2-2007/002598
- DE-U1-202007 007 921
- DE-U1-202008 002 931
- US-A- 5 603 318
- US-A1- 2004 133 189

## Beschreibung

Die vorliegende Erfindung betrifft ein Gerät zum Behandeln des menschlichen oder tierischen Körpers durch mechanische Druckwellen.

Solche Vorrichtungen sind an sich bereits bekannt, insbesondere aus dem Bereich der Lithotripsie. Dort werden mit fokussierten mechanischen Druckwellen Körperkonkremente, insbesondere Steine im Körpergewebe, zertrümmert. Neben der Erzeugung durch elektrische Entladungen in Wasser sind auch Geräte entwickelt worden, die mechanische Druckwellen durch das Aufeinanderprallen eines beschleunigten Schlagtells und eines Prallkörpers erzeugen und mit Hilfe des Prallkörpers In Körpergewebe einkoppeln. Solche Geräte sind sowohl in der Lithotripsie mit einem direkten Kontakt zwischen dem Prallkörper bzw. einer mit dem Prallkörper verbundenen Sonde und dem Stein als auch bei anderen Behandlungen von biologischen Körpersubstanzen eingesetzt worden. Insbesondere sind hier die Behandlung von Muskelerkrankungen und von Erkrankungen im Übergangsbereich zwischen Muskeln und Knochen zu nennen.

Ein Beispiel für ein Gerät, das zu dem zuletzt genannten Typ zu rechnen ist, ist die in der EP 0 991 447 dargestellte Vorrichtung. Bei dieser sollen unfokussierte Druckwellen in das Körpergewebe eingekoppelt werden.

Bei solchen Geräten kann in vielen Fällen die Heftigkeit der eingekoppelten Druckwelle durch Einstellung eines Druckwerts einer pneumatischen Versorgungseinrichtung verändert werden. Je höher der anstehende Pneumatikdruck, umso heftiger wird das Schlagteil beschleunigt und umso größer ist der Impuls- und Energieübertrag auf den Prallkörper.

Überdies erlauben viele Geräte die Einstellbarkeit der Wlederholfrequenzen pneumatischer Pulse und damit der Wiedertlolfrequenz der Schläge des Schlagteils auf den Prallkörper und der daraus resultierenden eingekoppelten Druckwellen.

Im Übrigen erlauben viele Geräte den Austausch von Prallkörpern gegen hinsichtlich Geometrie und/oder Masse abweichende andere.

Die DE 20 2008 002 931 U1 zeigt ein Gerät nach dem Oberbegriff des Oberbegriff des Anspruchs 1 mit einem Schlagteil zum Aufprall auf einen Prallkörper, der aus gefärbter und damit leicht unterscheidbarer Keramik bestehen kann. Verschiedene Prallkörper können hinsichtlich Form und Masse unterschiedlich sein.

Die US 5,603,318 A zeigt eine Anlage zur Lokalisierung eines chirurgischen Instruments mittels zweier Kameras und Bildverarbeitungssoftware. Als Zusatzfunktion kann das chirurgische Instrument anhand seiner Kontur und/oder Farbe erkannt werden.

Die EP 1 504 724 A1 und die US 2004/133189 A1 zeigen invasive chirurgische Geräte mit verschiedenen Instrumentenaufsätzen und Ultraschallbeaufschlagung. Der Typ eines Instrumentenaufsatzes wird erkannt, um die richtige Resonanzfrequenz bzw. richtig angepasste Energieversorgung bereitzustellen.

Der vorliegenden Erfindung liegt das technische Problem zugrunde, eine hinsichtlich des Einsatzes des Prallkörpers verbesserte Vorrichtung, eine vorteilhafte Verwendung derselben und ein vorteilhaftes Verfahren unter Verwendung der Vorrichtung anzugeben.

Die Erfindung richtet sich auf ein Gerät zur Behandlung des menschlichen oder tierischen Körpers durch mechanische Druckwellen mit einem bewegbaren Schlagteil und einem Prallkörper, wobei das Gerät dazu ausgelegt ist, die Druckwellen durch Beschleunigen und Aufprallen des Schlagteils auf den Prallkörper zu erzeugen, gekennzeichnet durch eine Einrichtung zur automatischen Erkennung von Eigenschaften des Prallkörpers, und dadurch, dass das Gerät dazu ausgelegt ist, hinsichtlich des Typs des erkannten Prallkörpers geeignete Betriebsparameter für die Behandlung selbsttätig einzustellen und anhand der Erkennung des Prallkörpers die Einhaltung einer maximalen Betriebszahl des Prallkörpers zu überwachen.

Die Erfinder schlagen vor, das erfindungsgemäße Gerät so auszustatten, dass bestimmte Eigenschaften des verwendeten Prallkörpers von dem Gerät erkannt werden kann. Dies kann seinen Typ oder bestimmte technische Eigenschaften, also in irgendeiner Weise eine Prallkörpergattung, aber auch eine Seriennummer oder eine ähnliche individuelle Kennzeichnung betreffen. Bei der Erkennung der Prallkörpergattung kann es beispielsweise um die Unterscheidung von Applikatoren hinsichtlich ihrer geometrischen Form, insbesondere ihrer Austrittsgrenzflächenwölbung (flach, konvex oder konkav und in welchem Maße gewölbt), oder hinsichtlich ihrer Materialien (Härte, Schallleitungseigenschaften) oder auch ihrer Massen gehen.

Aus der Applikatormasse beispielsweise ergeben sich zusammen mit den Eigenschaften einer etwaigen elastischen Aufhängung des Prallkörpers die Schwingungseigenschaften bei einer axialen Auslenkung des Prallkörpers nach Auftreffen des Schlagteils. Die Prallkörpererkennung kann auch hinsichtlich dieser elastischen Aufhängung Aussagekraft haben, etwa indem unterschiedlich breite Elastomerringe Verwendung finden, für die bei unterschiedlichen Prallkörpern unterschiedlich breite Aufnahmenuten vorgesehen sind.

Eine solche Erkennung kann die Anzeige des Prallkörpertyps an dem Gerät zur Folge haben, sodass die Behandlungsperson eine Kontrollmöglichkeit oder eine Information zur Einstellung passender Bedienungsparameter hat. Das Gerät ist erfindungsgemäß dazu vorgesehen, passende Bedienungsparameter selbstständig einzustellen, oder zumindest die eingestellten Parameter auf Verträglichkeit mit dem Prallkörpertyp zu überprüfen.

Bei der Erkennung eines individuellen Prallkörpers, der also hinsichtlich seiner Individualität von einem bautypgleichen unterschieden werden kann, kann es beispielsweise um die Sicherstellung einer maximalen Zahl von Behandlungen oder Schlagteilkollisionen gehen. So können vorab Lebensdauerwerte in diesem Sinn vorgegeben werden und von dem Gerät insoweit sichergestellt werden, als bei Erreichen einer in dem Gerät gespeicherten maximalen Zahl ein erneuter Betrieb mit diesem Prallkörper verweigert wird oder eine Warnung angezeigt wird.

Beispielsweise kann in Zusammenhang mit dem erwähnten Aspekt der Einhaltung von Lebensdauerwerten auch eine Erkennung von in dem Prallkörper hinterlegten Benutzungszahlen erfolgen. Beispielsweise kann der Prallkörper einen elektronischen Speicher enthalten, der drahtlos umgeschrieben werden kann. So könnte das erfindungsgemäße Gerät bei der Benutzung des Prallkörpers die bereits mit diesem Prallkörper erfolgten Behandlungen erfassen und überwachen und die Einhaltung einer diesbezüglichen Begrenzung sicherstellen, etwa indem der Speicherinhalt mit jeder Behandlung um eins erhöht wird und bei Erreichen einer Grenze der aus dem Speicher ausgelesenen Behandlungszahl der weitere Betrieb verweigert wird oder eine Warnung angezeigt wird. Die Erfindung richtet sich also auf austauschbare Prallkörper, wobei der eingesetzte Prallkörper von anderen unterscheidbar ist, also eine eigentliche Erkennung des Prallkörpers selbst stattfindet, sei es hinsichtlich seines technischen Typs oder seiner Gattung oder auch seiner Identität im Sinne einer Serien nummer.

Die Festlegung einer bestimmten Benutzungsdauer oder maximalen Behandlungszahl für einen Prallkörper kann auch mit den eingesetzten Benutzungsparametern kombiniert werden und dabei die Verschleißträchtigkeit der eingestellten Parameter berücksichtigen. Beispielsweise können so bei geringerem Druck größere Schlagzahlen zugelassen werden. Dies kann durch die Gerätesteuerung berücksichtigt werden und eine differenziertere Ausnutzung der verfügbaren komplikationsfreien Lebensdauer eines Prallkörpers ermöglichen. Gegenüber einer nicht gerätetechnisch gestützten Beachtung durch den Benutzer, der ja nur die Zahl der Behandlungen mitnotieren könnte, ergeben sich hier also weitergehende Vorteile.

Einen Sonderfall können Einmalprallkörper darstellen, die beispielsweise aus Hygienegründen und wegen geringer Stückkosten (z. B. bei Kunststoffformteilen) verwendet werden können. Hier könnte das Gerät sicherstellen, dass ein einmal (jedenfalls mit diesem Gerät oder mit damit datentechnisch verbundenen Geräten schon einmal) benutzter Prallkörper nicht erneut benutzt werden kann, etwa weil die Behandlungsperson unzulässigerweise sparen will.

Schließlich kann das Gerät anhand einer Prallkörpererkennung auch prüfen, ob der eingesetzte Prallkörper überhaupt für das Gerät geeignet ist, und ggf. den Betrieb verweigern oder eine Warnung abgeben.

Grundsätzlich kann die Erfindung ausgeführt werden, ohne den Prallkörper hierzu in besonderer Weise auszustatten. Beispielsweise könnte die Erkennungseinrichtung unterschiedliche magnetische Eigenschaften metallischer Prallkörper mit ausreichend unterschiedlichen Formen und/oder Metallmaterialien ermitteln und unterscheiden, etwa über eine Induktivitätsmessung, also durch Einsatz einer in dem übrigen Behandlungsgerät angebrachten elektromagnetischen Spule. Allgemeiner gesprochen können elektromagnetische Eigenschaften wie die elektrische Leitfähigkeit des Prallkörpers zur Erkennung dienen. Dies gilt natürlich auch für andere Eigenschaften, die der Prallkörper ohnehin hat, etwa für die Masse.

Bevorzugt ist allerdings eine Markierung des Prallkörpers, also eine zu dem Zweck der Erkennung vorgesehene Gestaltung oder Einrichtung. Dabei soll im Folgenden eine Befestigungseinrichtung für den Prallkörper, etwa die Applikatorkappe der Ausführungsbeispiele, im Sinne der Markierung dann als Prallkörperteil betrachtet werden, wenn sie beim Austauschen des Prallkörpers mit diesem gemeinsam ausgetauscht wird, ihm also zugeordnet ist. Hinsichtlich der Funktion der Erkennung läuft dann eine Erkennung einer dem Prallkörperzugeordneten Befestigungseinrichtung und eine Markierung des Prallkörpers selbst auf das Gleiche hinaus. In diesem Sinn kann der Begriff des Prallkörpers im Folgenden also auch eine Einheit aus Prallkörper und Befestigungseinrichtung meinen, wobei die Markierung in der Befestigungseinrichtung liegen kann.

Eine erfindungsgemäße Gruppe von Markierungen verfügt über zumindest einen elektrischen Kontakt, etwa um einen elektrischen Widerstand des Prallkörpers oder an dem Prallkörper messen zu können. Dieser Widerstand kann beispielsweise ein zusätzlicher Leiter sein, der außen an dem Prallkörper angebracht ist, etwa ein über einen Abschnitt gelegter Leiterstreifen. Im Ausführungsbeispiel wird ein ringförmiger Leiterstreifen dargestellt, der über einen Umfangsabschnitt des Prallkörpers verläuft. Eine andere Gruppe von Markierungen wird über Licht im weitesten Sinn ausgelesen, also einschließlich Infrarotstrahlung. Beispielsweise kann über Licht ein Code ausgelesen werden, der aus einer bestimmten Folge von mehr oder weniger reflektierenden Flächen besteht, im Falle von sichtbarem Licht etwa ein aus hellen und dunklen Flächen bestehender Streifen, insbesondere ein sog. Barcode.

Insbesondere bei der Erkennung von Markierungen über Licht, aber auch unabhängig davon, kann es notwendig oder gewünscht sein, den Prallkörper in einer bestimmten Orientierung hinsichtlich seiner Längsachse einzubauen. Zur Vorgabe einer solchen Orientierung können Formschlusselemente dienen, also beispielsweise vorspringende Teile, die in an dem Prallkörper eingebrachte Nuten eingreifen. Dann kann der Prallkörper nur in einer bestimmten gewünschten Orientierungen (oder in einer Mehrzahl jeweils geeigneter Orientierungen) angebracht werden.

Eine andere Möglichkeit besteht darin, einen Magneten, etwa in dem Prallkörper, und einen dazu passenden Magnetsensor im übrigen Gerät vorzusehen, oder auch umgekehrt.

Zur Auslesung über Licht, also zur optischen Auslesung, kann ein optischer Leiter vorgesehen sein, der vorzugsweise zwischen einer Außenhülse des Gerätes und einem Innenrohr, das zum Führen des Schlagteils dient, verlaufen kann. Zur Veranschaulichung wird auf die Ausführungsbeispiele verwiesen.

Eine weitere Möglichkeit zur Erkennung von Markierungen besteht in der Verwendung von elektromagnetischen Wellen, etwa Radiofrequenzwellen. Hierzu kann die Markierung einen Transponder aufweisen und die Erkennungseinrichtung eine Empfangs/Sendespule, die den Typ des Transponders feststellen kann. Die Empfangs/Sendespule kann beispielsweise zwischen dem bereits erwähnten Innenrohr und der bereits erwähnten Außenhülse angeordnet sein, wozu wieder auf die Ausführungsbeispiele verwiesen wird. Die Empfangs/Sendespule kann aber auch in einem Basisgerät untergebracht sein, das zur Versorgung eines mobilen Handgerätes dient und mit dem Handgerät über eine Leitung verbunden ist. In diesem Fall kann es notwendig sein, den Prallkörper vor der Montage oder das Handgerät mit dem bereits darin montierten Prallkörper in die Nähe des Basisgerätes zu bringen, um die Erkennung der Markierung zu ermöglichen, also das Auslesen des Transponders.

In diesem Sinn gehört eine Mehrzahl verwendbarer und gegeneinander austauschbarer Prallkörper zum bevorzugten Ausstattungsumfang des erfindungsgemäßen Gerätes, wenngleich die Erfindung auch schon dadurch verwendet werden kann, dass jeweils nur ein Prallkörper benutzt wird, aber beim Ersetzen desselben, also beim dauerhaften Umrüsten des Behandlungsgerätes, dennoch der neue Prallkörper erkannt werden soll. Die bevorzugte Ausstattung des Behandlungsgerätes mit einer Mehrzahl Prallkörper betrifft natürlich auch den bereits angesprochenen Fall von Einmalprallkörpem oder solche mit sehr begrenzter Lebensdauer, die gewissermaßen als Verbrauchsartikel auf Vorrat vorhanden sind.

Der Prallkörper kann übrigens auch mehrteilig aufgebaut sein und insbesondere auch mit einer Kappe oder einer ähnlichen Einrichtung versehen sein, die bei der Verwendung zwischen dem Prallkörper und dem behandelten Körper liegt, sei sie nun aus hygienischen Gründen vorgesehen, zur Impedanzanpassung oder zur Vermeidung von Verletzungen oder Beeinträchtigungen der Haut. Die Markierung bzw. Erkennung betrifft jedenfalls die Einheit einer Mehrzahl von Teilen, soweit sie als solche austauschbar und von anderen Einheiten unterschieden werden muss.

Die Prallkörpererkennung kann insbesondere dazu führen, dass ein erkannter Typ des Prallkörpers angezeigt wird. Damit hat ein Nutzer die Möglichkeit, den Prallkörpertyp zu kontrollieren und/oder bestimmte Bedienungsparameter, Geräteparameter oder andere Umstände der Nutzung darauf abzustimmen.

Überdies ist das Gerät selbstständig zur Einstellung geeigneter Betriebsparameter für den geeigneten Prallkörpertyp ausgelegt. Insbesondere kann das Gerät auch dazu ausgelegt sein, die Einhaltung einer maximalen Betriebszahl des Prallkörpers, die ebenfalls als Betriebsparameter betrachtet wird, zu überwachen und durch Signalanzeige oder Nichtinbetriebnahme des Prallkörpers zu verhindern, dass eine vorgegebene maximale Betriebszahl überschritten wird.

Schließlich ist die Verwendung der Vorrichtung gerade bei der Behandlung von Körperweichgewebe, beispielsweise Muskeln oder Sehnen, bevorzugt. Dies schließt die Behandlung knochennaher Bereiche und eine Stoßwellenakupunktur ein. Typische Indikationen sind Ansatztendinosen und andere Anwendungen in Orthopädie und Chirurgie wie Kalkschultern, Fersenschmerzen, Pseudarthrosen, aber auch Muskelzerrungen. Weitere Indikationen gibt es in der Neurologie, etwa die Verbesserung der Motorik nach Schlaganfällen, die Behandlung von Spasmen nach Traumen sowie Poly-Neuropathien. In der Urologie können etwa chronische Beckenbodenschmerzen behandelt werden; in der Angiologie / Dermatologie und Chirurgie außerdem Narben oder Hautverbrennungen behandelt sowie eine Verbesserung der Wundheilung erzielt werden.

Die Erfindung wird im Folgenden anhand einiger Ausführungsbeispiele näher erläutert, wobei die einzelnen Merkmale für alle genannten Anspruchskategorien und auch in anderen als den dargestellten Kombinationen erfindungswesentlich sein können.
- Figur 1: zeigt ein erfindungsgemäßes Gerät im Längsschnitt, wobei Einzelhei- ten der Erfindung in Figur 1 nicht dargestellt sind.
- Figuren 2-9: zeigen jeweils einen Ausschnitt aus Figur 1 mit zusätzlich dargestell- ten Erkennungseinrichtungen und/oder Markierungen, und damit ein erstes bis achtes Ausführungsbeispiel.

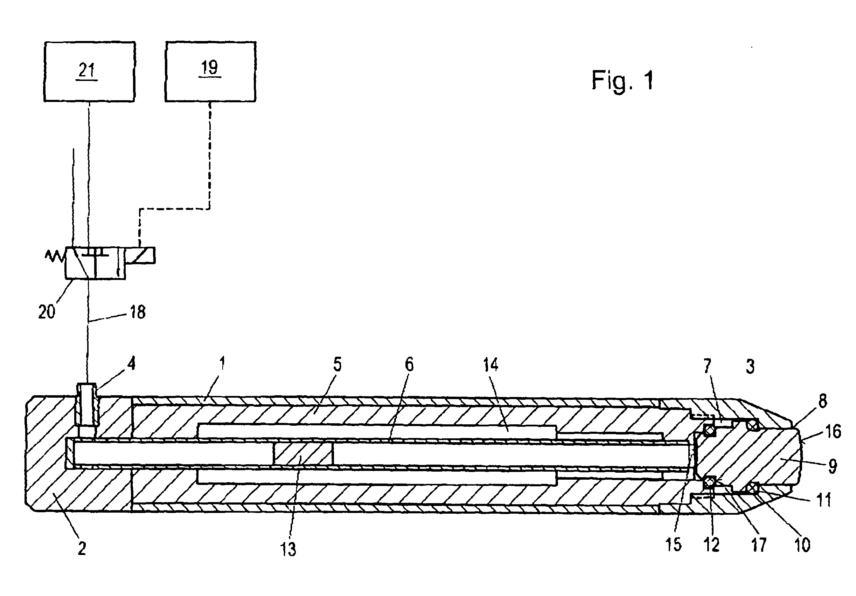

In Figur 1 ist in einem Schnitt entlang einer Längsachse eine erfindungsgemäße Vorrichtung zur Einkopplung von fokussierten mechanischen Druckwellen in beispielsweise den menschlichen Körper dargestellt. Ein Rohrstück bildet eine Außenhülse 1, die von einer in der Anwendung körperfernen Zuluftkappe 2 und einer in der Anwendung körperzugewandten Applikatorkappe 3 jeweils endseitig abgeschlossen ist.

Die Zuluftkappe 2 enthält einen Druckluftanschluss 4 für eine pneumatische Versorgung. In an sich bekannter Weise ist an diesen Druckluftanschluss 4 über eine pneumatische Versorgungsteitung ein von einer Ansteuereinheit 19 gesteuertes Ventil 20, insbesondere Magnetventil, angeschlossen, das in einem gleich bleibenden iterativen Takt zwischen etwa 1 Hz und 50 Hz Druckluftpulse über den Druckluftanschluss einkoppelt.

Die Vorrichtung ist als mit der Hand einer Bedienungsperson zu haltendes Gerät ausgebildet, das über die erwähnte Pneumatikleitung 18 an eine Basisstation mit der Ansteuereinheit 19 und dem Kompressor 21 angeschlossen ist und auf den Patienten manuell aufgesetzt werden kann. Es dient zur Behandlung von Welchgewebe, insbesondere Muskeln.

Die Einzelheiten der Pneumatikversorgung sind nicht Gegenstand dieser Erfindung und dem Fachmann aus dem Stand der Technik geläufig. Vorzugsweise ist die Frequenz einstellbar. Der iterative Betrieb kann komplizierter als mit einer einfachen gleich bleibenden Wiederholung von Pulsen einer bestimmten Frequenz erfolgen, insbesondere auch mit einer Mehrzahl in relativ kurzem Zeitabstand, also mit einer relativ hohen Frequenz, aufeinander folgenden Schlägen, wobei Gruppen von Schlägen in diesem kürzeren Zeitabstand durch etwas größere Zeitabstände voneinander getrennt sind. Die Einzelheiten hierzu sind nicht Gegenstand der vorliegenden Erfindung, können aber damit kombiniert sein.

In der Außenhülse 1 ist über einen Einsatz 5 ein Führungsrohr 6 gehalten, dessen bei der Anwendung körperfemes Ende in der Zuluftkappe 2 endet und dort mit dem Druckluftanschluss 4 kommuniziert. Das in der Anwendung körperseitige Ende des Führungsrohres 6 endet in einem Teil des Einsatzes 5, der in die Applikatorkappe 3 hineinragt, und zwar kurz vor dem dortigen Ende des Einsatzes 5 und einem Innenraum 7 in der Applikatorkappe 3.

In dem Innenraum 7, der mit zwei radialen Schultern in eine in der Anwendung körperseitige Applikatoröffnung 8 übergeht, ist ein Prallkörper 9 aufgenommen. Dieser stützt sich über einen O-Ring 10 aus einem Elastomer an einer der radialen Schultern ab und weist hierzu einen Flansch 11 auf. Ein zur körperfernen Seite gerichtetes Ende 15 des Prallkörpers 9 stützt sich über einen weiteren O-Ring 12 an dem Einsatz 5 ab, und zwar an einer das bereits erwähnte Ende des Einsatzes 5 umgebenden Stirnfläche. Dabei liegt der O-Ring 12 zwischen dieser Stirnfläche und einem Flansch 17 bzw. einer Schulter des Prallkörpers 9. Die Applikatoröffinung 8 dient dabei zu einer in der Längsrichtung verschiebbaren Führung des Prallkörpers 9 und fixiert diesen quer zur Längsrichtung. Die Axialverschiebbarkeit ist durch die Nachgiebigkeit der Elastomerringe 10 und 12 begrenzt und liegt bei in Luft betriebener Vorrichtung relativ zur Restvorrichtung deutlich über 0.6 mm.

Auf die Merkmale des Prallkörpers 9, der hier gleichzeitig den auf die Haut aufzusetzenden Applikator bildet, wird im Folgenden noch näher eingegangen. Er Ist durch Abschrauben der Applikatorkappe 3 austauschbar.

In dem angrenzenden Bereich des Führungsrohres 6 ist ein in Figur 1 mit dem Prallkörper 9 in Kontakt stehendes Schlagteil 13 eingesetzt. Dieses passt (in Bezug auf das Führungsrohr und die im Wesentlichen zylindrische Geometrie des Schlagteils 13) radial mit geringem Spiel. Das Schlagteil 13 kann durch Druckunterschiede der Luftsäule in dem Führungsrohr 6 vor und hinter ihm (d. h. in Figur 1 rechts und links des Schlagteils 13) in dem Führungsrohr hin- und herbewegt werden und insbesondere auf den Prallkörpers 9 zu beschleunigt werden. Hierzu wird es aus einer Ausgangsposition (nicht gezeigt) In Figur 1 links durch einen Druckluftstoß durch den Druckluftanschluss 4 beschleunigt und trifft mit seiner dem Prallkörper 9 zugewandten Frontfläche (in Figur 1 der Übersichtlichkeit halber nicht bezeichnet) auf den Prallkörper 9 auf.

Die Rückbewegung des Schlagteils 13 erfolgt zusätzlich zu einem Zurückprallen nach der Kollision durch ein Rückströmen der Luft aus einer das Führungsrohr 6 innerhalb des Einsatzes 5 umgebenden Staukammer 14. In diese wird die Luft bei der Beschleunigung des Schlagteils 13 in Richtung zu dem Prallkörper 9 verdrängt und damit dort komprimiert. Wenn das Magnetventil 20 in der Pneumatikzuleitung 18 des Druckluftanschlusses 4 den Druck wegschaltet, wird das Schlagteil 13 damit in die Ausgangsstellung zurückbewegt. Dies kann natürlich auch durch eine zusätzliche oder alternative Druckbeaufschlagung der Staukammer 14 oder eines anderen Luftvolumens körperseitig von dem Schlagteil 13 erfolgen. Das in der Anwendung körperfeme Ende des Führungsrohres 6 endet in einem Magnethalter 17 für das Schlagteil 13.

Der Prallkörper 9 hat eine rotationssymmetrische Zylinderform und ist in axialer Richtung durch die Eintrittsfläche 15 und die etwas konvexe Austrittsfläche 16 begrenzt. Der Außenmantel weist die bereits beschriebenen flanschartigen Strukturen 11 und 17 auf, die Anlageschultem für die O-Ringe 10 und 12 bilden. Im Übrigen ist ein austrittsseitiger Teil der Zylindergeometrle mit konstantem Radius gestaltet und damit in der Öffnung 8 axial verschiebbar.

Prallkörper wie der hier gezeichnete Prallkörper 9 können sich hinsichtlich Form, Material und Lagerung erheblich unterschieden. So gibt es unterschiedliche fokussierende und nicht fokussierende Formen, vergleiche etwa auch die Rotationsellipsoidform in der DE 10 2007 013 288, und verschiedene gewölbte Austrittsflächen im Stand der Technik. Ferner kommen verschiedene Materialien in Betracht, etwa Edelstahl, Titan, und verschiedene Keramiken wie Siliziumnitrid sowie Kunststoffe. Schließlich können verschiedene Prallkörper unterschiedlich hart gelagert sein und dabei unterschiedliche Hübe ausführen, also eine unterschiedlich lange makroskopische Bewegung bei der Einkopplung der Druckwelle durchführen.

Verschiedene Ausgestaltungen in diesem Sinn sind jeweils für bestimmte Anwendungen besonders gut geeignet, etwa für Akupunktur, Sehnenansatzbehandlungen, Muskelbehandlungen, Triggerpunktbehandlungen etc.. Hierbei werden verschiedene Parameter für beispielsweise den Antriebsdruck oder die Pulsfrequenz eingesetzt, die von dem verwendeten Prallkörper und der Indikation abhängen. Im Sinne einer sinnvollen Systemsteuerung ergibt sich daher ein erheblicher Vorteil, wenn eine automatische Erkennung des Prallkörpertyps durchgeführt wird.

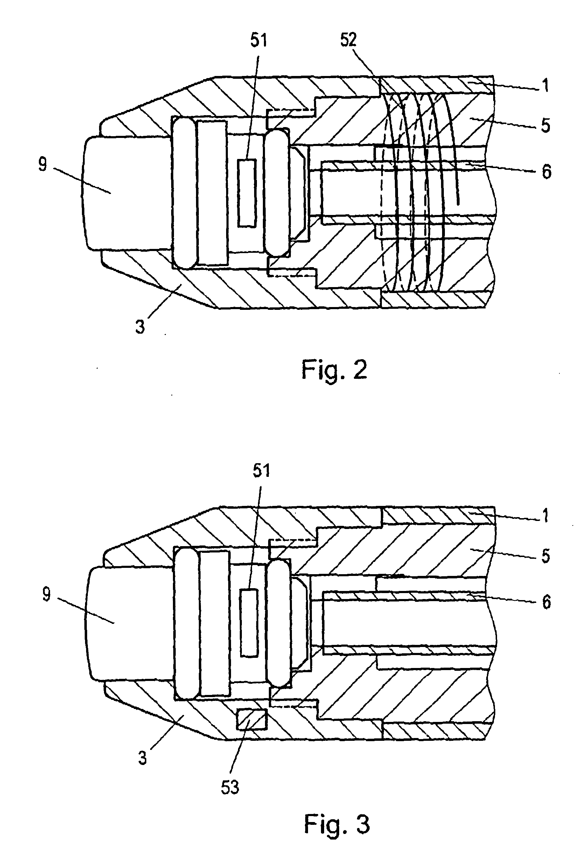

Figur 2 zeigt ein erstes Ausführungsbeispiel als (seitenverkehrten) Ausschnitt aus Figur 1 mit zusätzlich eingezeichneten Einzelheiten zur Erfindung. Figur 2 zeigt einen sogenannten RFID-Transponder 51, der auf dem Prallkörper 9 angebracht ist. Mit 52 ist eine nur symbolisch angedeutete Empfangs/Sendespule In dem Handteil aus Figur 1 dargestellt. Diese ist zwischen der Außenhülse 1 und dem Einsatz 5 und dabei möglichst nah an dem Prallkörper 9 und dem daran montierten RFID-Transponder 51, also möglichst weit links in Figur 2, montiert. Man erkennt in der Figur, dass beim Abnehmen der Applikatorkappe 3 der Bereich, in dem die Empfangs/Sendespule 52 angebracht ist, dennoch nicht geöffnet wird und diese insoweit nicht gefährdet ist. Durch die Nähe erleichtert sich die Erkennung des RFID-Transponders 51. Insbesondere werden Kopplungen zu anderen Prallkörpern, die beispielsweise auf einem Tisch im Behandlungszimmer in der Nähe des Handstücks liegen könnten, in dieser Weise möglichst schwach relativ zu der Kopplung an den dargestellten RFID-Transponder 51 gehalten.

RFID-Erkennungssysteme sind an sich bekannt. Über ein elektromagnetisches Hochfrequenzfeld mit typischerweise 13,56 MHz wird der RFID-Transponder 51 mit Energie versorgt und ausgelesen. Da hier nur kurze Reichweiten erforderlich sind, benötigt das System nur geringe Leistungen. Ferner kann der RFID-Transponder 51 sehr klein sein und beeinträchtigt die Eigenschaften des Prallkörpers 9 nicht.

In Figur 3 ist in analoger Weise ein zweites Ausführungsbeispiel dargestellt. Auch hier ist ein RFID-Transponder 51 vorgesehen, wobei jedoch die Empfangs/Sendespule bei diesem zweiten Ausführungsbeispiel in dem nicht dargestellten Basisgerät, vgl. Figur 1, enthalten ist. Bei solchen Ausgestaltungen der Erfindung muss der Nutzer also grundsätzlich mit dem noch nicht eingebauten einzusetzenden Prallkörper 9 oder auch dem eingebauten Prallkörper 9, also dem körperseitigen Ende des Handstücks, in die Nähe des Basisgeräts kommen, um eine Erkennung zu ermöglichen. Bei diesem zweiten Ausführungsbeispiel ist allerdings eine zusätzliche Verbesserung insoweit vorgesehen, als die Gefahr ausgeschlossen werden soll, dass versehentlich ein in der Nähe des Basisgerätes befindlicher Prallkörper, der aber gar nicht zum Einsatz vorgesehen ist, erkannt wird. Dazu verfügt dieses Ausführungsbeispiel über einen Permanentmagneten 53 in der Applikatorkappe 3, die den Prallkörper 9 an dem Handstück hält. Der RFID-Transponder 51 kann die Anwesenheit des Permanentmagneten 53 erkennen und damit den eingebauten Zustand des Prallkörpers 9 von einem nicht eingebauten Zustand unterscheiden. Der RFID-Transponder wird gewissermaßen durch die Detektion des Permanentmagneten 53 freigeschaltet.

In diesem Zusammenhang kann es von Vorteil sein, den RFID-Transponder 51 nicht wie gezeigt, von dem Permanentmagneten unabhängig an dem Prallkörper 9 zu montieren, sondern an dem Randbereich, der eine möglichst große räumliche Nähe zwischen dem Permanentmagneten 53 und dem RFID-Transponder 51 zu gewährleistet. Dazu kann ein nicht dargestellter Formschluss dargestellt sein, etwa eine Nut in dem Prallkörper 9 und eine passende Nase in der Applikatorkappe 3, um bei der Montage des Prallkörpers 9 die drehsinnrichtige Position zu gewährleisten und eine versehentliche Anordnung des RFID-Transponders 51 in einer anderen als der dem Permanentmagneten 53 angenäherten Winkelposition zu verhindern.

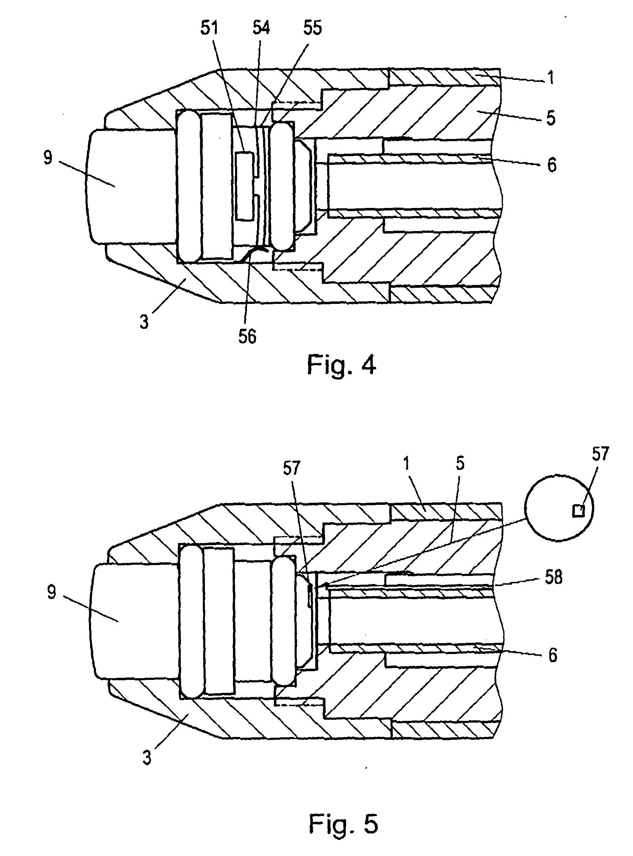

Figur 4 zeigt wieder den RFID-Transponder 51 aus den Figuren 2 und 3. Dieser ist hier allerdings über Leitungen an zwei Ringelektroden 54 und 55 angeschlossen, die über einen in Figur 4 im unteren Bereich dargestellten Federkontakt 56 in der Applikatorkappe 3 kontaktiert werden können. Der Federkontakt 56 kann die beiden Ringelektroden 54 und 55 kurzschließen und damit in einer ähnlichen Weise wie beim zweiten Ausführungsbeispiel aus Figur 3 den RFID-Transponder 51 des montierten Prallkörpers 9 von anderen unterscheidbar machen. Im Übrigen gelten die Erläuterungen zum zweiten Ausführungsbeispiel.

Das nächste vierte Ausführungsbeispiel in Figur 5 verwendet eine optisch erkennbare Markierung des Prallkörpers 9, nämlich einen zweidimensionalen Barcode 57. Dieser ist an einer körperabgewandten Stirnfläche des Prallkörpers 9 exzentrisch angebracht, wie der kleine Ausschnitt rechts neben Figur 5 zeigt, und über ein Glasfaserbündel 58, dass an seinem markierungszugewandten Ende gewissermaßen einen Lesekopf bildet, erkennbar. Dazu kann das Glasfaserbündel 58 über eine nicht dargestellte Lichtquelle, etwa eine LED oder Laserdiode beleuchtet sein. Insbesondere kann der Barcode, statt in einem Zug, quasi parallel, als Bild erfasst und dann elektronisch ausgewertet zu werden, auch quasi seriell durch Abscannen unter Verwendung der Mehrzahl Glasfasern (also der Glasfasern der Reihe nach) ausgelesen werden.

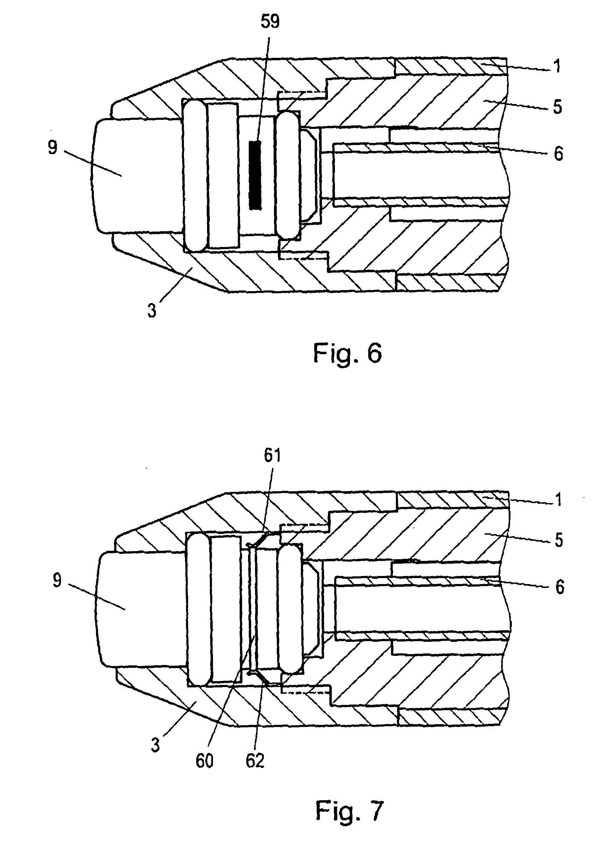

Figur 6 zeigt das nächste Ausführungsbeispiel, bei dem der Prallkörper 9 einen auf seiner Mantelfläche angebrachten Barcode 59 aufweist. Dieser Barcode ist in Figur 6 nur symbolisch als schwarzer Streifen 59 dargestellt. Er ist dazu vorgesehen, vor Montage des Prallkörpers 9 durch ein Lesegerät erkannt zu werden, das in dem bereits mehrfach erwähnten Basisgerät eingebaut ist. Hier kommt es also darauf an, dass der Benutzer sicherstellt, tatsächlich den erkannten Prallkörper 9 und keinen anderen zu montieren und, bereits als Voraussetzung hierfür, beim Wechseln eines Prallkörpers 9 tatsächlich auf eine neue Erkennung zu achten.

Das sechste Ausführungsbeispiel in Figur 7 ist nicht auf eine optische Erkennung sondern eine elektrische gerichtet. Hierzu ist ein auf derselben Umfangsfläche des Prallkörpers 9 wie im fünften Ausführungsbeispiel umlaufender Widerstandsstreifen 60 vorgesehen, der über Kontaktfedern 61 und 62 kontaktiert werden kann. Durch unterschiedliche elektrische Widerstandswerte des Widerstandsstreifen 60 zwischen den Abgriffsstellen der Federkontakte 61 und 62 lassen sich unterschiedliche Prallkörpertypen erkennen und unterscheiden. Bei einer nicht dargestellten Ausführungsform könnten in dieser Weise auch Speicher wie beispielsweise ein EEPROM eingesetzt werden, die durch entsprechende Kontakte ausgelesen werden können.

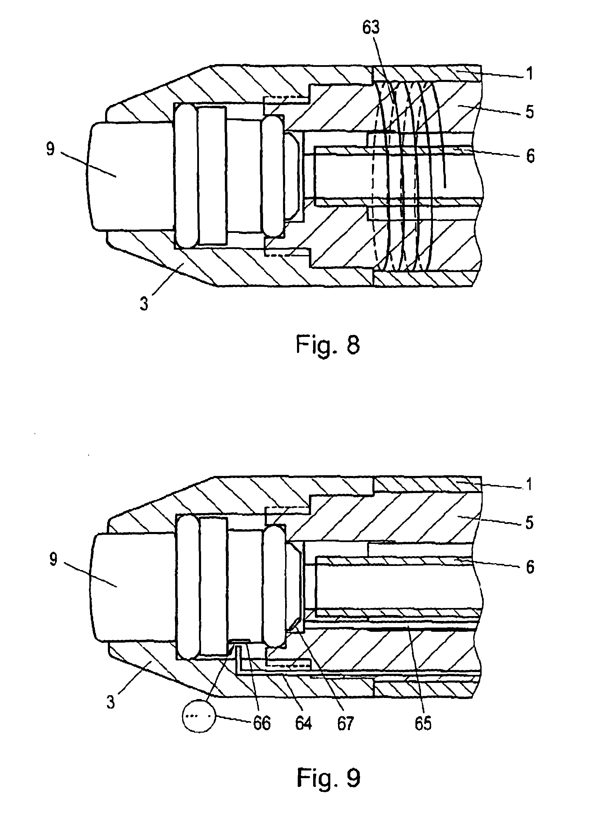

Figur 8 zeigt mit dem siebten Ausführungsbeispiel wieder eine Spule, hier allerdings eine Detektorspule 63. Sie dient zur induktiven Bestimmung des Prallkörpertyps durch Ermittlung seiner magnetischen Impedanz. Dies setzt ausreichend deutliche Unterschiede zwischen den in Frage kommenden Prallkörpertypen hinsichtlich Material und/oder Größe und/oder Form voraus. Für die Montage der Detektorspule 63 gelten die Erläuterungen zur Empfangs/Sendespule 53 des ersten Ausführungsbeispiels in Figur 2.

Das letzte Austührungsbeispiel in Figur 9 bezieht sich wieder auf eine optische Erkennung wie in Figur 5, also bei dem vierten Ausführungsbeispiel .

Hier sind zwei Lichtleiter 64 und 65 vorgesehen, ähnlich wie beim vierten Ausführungsbeispiel in Figur 5. Belde sind lichtwellenleitende Glasfaserbündel. Der Lichtleiter 64 läuft in seinem letzten Stück durch die Applikatorkappe 3 und weist also eine nicht näher dargestellte optische Koppelstelle zwischen Applikatorkappe 3 und Einsatz 5 auf.

Der zweite Lichtleiter 65 läuft weiter innen und ähnlich wie der Lichtleiter 58 aus Figur 5. Hier werden Punktfolgen als Markierung abgetastet, die beispielhaft bei der Markierung 66 dargestellt sind, und bei der dem anderen Lichtleiter 65 zugeordneten Markierung 67 in entsprechender Weise an der Fase des Prallkörpers 9 vorhanden sind. Dabei bilden die beiden Markierungen und die beiden Lichtleiter Alternativen, die aber auch in Kombination vorgesehen sein können, und verdeutlichen die unterschiedlichen geometrischen Möglichkeiten, insbesondere die Anbringung auf einer Mantelfläche wie bei der Markierung 66, die Anbringung auf einer schrägen Fläche wie bei der Markierung 67 (und natürlich die Anbringung an einer Stirnfläche wie 57 in Figur 5). Beispielsweise können die dargestellten vier schwarzen Punkte zusammen mit der Lücke ein Startbit und weitere vier Informationsbits darstellen. Auf das Startbit kann auch verzichtet werden, wenn die drehsinngenaue Montage des Prallkörpers 9 ausreichend präzise funktioniert. Dazu können die bereits erläuterten Formschlüsse dienen, auch in Form von Schrägen an dem Prallkörper 9, die nur eine bestimmte drehsinnrichtige Montage ermöglichen, weil sie Vorsprüngen an der Applikatorkappe 3 oder dem Einsatz 5 entsprechen.

## Patentansprüche

1. Gerät zur Behandlung des menschlichen oder tierischen Körpers durch mechanische Druckwellen mit
einem bewegbaren Schlagteil (13) und
einem Prallkörper (9),
wobei das Gerät dazu ausgelegt ist, die Druckwellen durch Beschleunigen und Aufprallen des Schlagteils (13) auf den Prallkörper (9) zu erzeugen,
**gekennzeichnet durch** eine Einrichtung (51 - 67) zur automatischen Erkennung von Eigenschaften des Prallkörpers (9),
und **dadurch**, dass das Gerät dazu ausgelegt ist, hinsichtlich des Typs des erkannten Prallkörpers (9) geeignete Betriebsparameter für die Behandlung selbsttätig einzustellen und anhand der Erkennung des Prallkörpers (9) die Einhaltung einer maximalen Betriebszahl des Prallkörpers (9) zu überwachen.

2. Gerät nach Anspruch 1, bei dem der Prallkörper (9) austauschbar ist und durch die Erkennung von anderen einsetzbaren Prallkörpern (9) unterschieden werden kann.

3. Gerät nach Anspruch 1 oder 2, bei dem der Prallkörper (9) eine durch die Erkennungseinrichtung (51 - 62, 64 - 67) erkennbare Markierung (51, 54, 55, 57, 59, 60, 66, 67) aufweist.

4. Gerät nach Anspruch 3, bei dem die Markierung (60) über einen elektrischen Kontakt (61, 62) erkennbar ist.

5. Gerät nach Anspruch 4, bei dem die Markierung ein elektrischer Widerstand (60) ist, insbesondere ein ringförmiger Leiterstreifen um den Umfang eines Abschnitts des Prallkörpers (9).

6. Gerät nach Anspruch 3, bei dem die Markierung (57, 66, 67) über Licht einschließlich Infrarotstrahlung erkennbar ist.

7. Gerät nach Anspruch 6, bei dem die Markierung (57, 66, 67) ein Hell/Dunkel-Code ist, insbesondere ein Barcode.

8. Gerät nach Anspruch 6 oder 7, bei dem der Prallkörper (9) und ein Teil des übrigen Geräts einen Magneten (53) und einen komplementären Magnetsensor (51) oder komplementäre Formschlusselemente zur Orientierungsvorgabe bei der Montage des Prallkörpers (9) aufweisen.

9. Gerät nach einem der Ansprüche 6 - 8, bei dem in dem Gerät ein optischer Leiter (58, 64, 65) zum Erkennen der Markierung integriert ist, und zwar zwischen einem Innenrohr (6) zum Führen des Schlagteils (13) und einer Außenhülse (1) des Geräts.

10. Gerät nach Anspruch 3, bei dem die Markierung (51) über Radiofrequenzwellen auslesbar ist und einen Transponder (51) und die Erkennungseinrichtung eine Empfangs/Sendespule (52) aufweist.

11. Gerät nach Anspruch 10, bei dem die Empfangs/Sendespule (52) zwischen einem Innenrohr (6) zum Führen des Schlagteils (13) und einer Außenhülse (1) des Geräts angeordnet ist.

12. Gerät nach Anspruch 10, bei dem in einem ein Handgerät des Geräts versorgenden Basisgerät eine Empfangs/Sendespule der Erkennungseinrichtung vorgesehen ist.

13. Gerät nach Anspruch 1, bei dem der Prallkörper (9) keine Markierung aufweist und die Erkennungseinrichtung (63) den Prallkörper anhand elektromagnetischer Eigenschaften erkennen kann, insbesondere anhand einer magnetischen Induktivitätsmessung.

## Claims

1. An apparatus for treating a human or animal body by mechanical shockwaves having
a moveable projectile (13) and
an impact body (9),
said apparatus being adapted to produce said shockwaves by accelerating and colliding said projectile (13) with said impact body (9)
**characterized by** a detection device (51 - 67) for an automatic detection of properties of said impact body (9)
and in that said apparatus is adapted to automatically set operation parameters adapted to a type of said detected impact body (9) for said treatment and to monitor a maximum operation number of said impact body (9) by means of said detection of said impact body (9).

2. The apparatus of claim 1 wherein said impact body (9) is exchangeable and can be distinguished from other impact bodies (9) being mountable by said detection.

3. The apparatus of claim 1 or 2 wherein said impact body (9) has a marking (51, 54, 55, 57, 59, 60, 66, 67) detectable by said detection device (51 - 62, 64 - 67).

4. The apparatus of claim 3 wherein said marking (60) is detectable via an electrical contact (61, 62).

5. The apparatus of claim 4 wherein said marking is an electrical resistor (60), in particular a ring-shaped conductor strip around a circumference of a portion of said impact body (9).

6. The apparatus of claim 3 wherein said marking (57, 66, 67) is detectable by light including infrared light.

7. The apparatus of claim 6 wherein said marking (57, 66, 67) is a bright/dark code, in particular a barcode.

8. The apparatus of claim 6 or 7 wherein said impact body (9) and a part of the remaining apparatus have a magnet (53) and a complementary magnetic sensor or have complementary form closing elements for defining an orientation in mounting said impact body (9).

9. The apparatus of one of claims 6 - 8 having an integrated optical conductor (58, 64, 65) for detecting said marking, mounted in said apparatus, namely between an inner tube (6) for guiding said projectile (13) and an outer casing (1) of said apparatus.

10. The apparatus of claim 3 wherein said marking (51) is readable by radio frequency waves and comprises a transponder (51) and said detection device comprises a receiving/transmitting coil (52).

11. The apparatus of claim 10 wherein said receiving/transmitting coil (52) is arranged between an inner tube (6) for guiding said projectile (13) and an outer casing (1) of said apparatus.

12. The apparatus of claim 10 having a receiving/transmitting coil (52) of said detection device arranged in a basic apparatus supplying a hand apparatus of said apparatus.

13. The apparatus of claim 1 wherein said impact body (9) has no marking and said detection device (63) detects said impact body by electro-magnetic properties, in particular by a magnetic inductivity measurement.

## Revendications

1. Appareil pour le traitement du corps humain ou animal, par des ondes de choc, avec
une pièce de butée mobile (13) et
un corps d'impact (9),
l'appareil étant conçu comme produire les ondes choc par l'accélération et l'impact de la pièce de butée (13) sur le corps d'impact (9),
**caractérisé par** un dispositif (51-67) pour la reconnaissance automatique des propriétés du corps d'impact (9),
et en ce que l'appareil est conçu pour régler automatiquement les paramètres de fonctionnement appropriés pour le traitement, selon le type du corps d'impact (9) reconnu, et pour surveiller l'observation d'un nombre d'exploitation maximal du corps d'impact (9), à l'aide de la reconnaissance du corps d'impact (9).

2. Appareil selon la revendication 1, dans lequel le corps d'impact (9) est interchangeable et peut être distingué par la reconnaissance d'autres corps d'impact (9) utilisables.

3. Appareil selon la revendication 1 ou 2, dans lequel le corps d'impact (9) comporte un repère (51, 54, 55, 57, 59, 60, 66, 67) susceptible d'être reconnu par le dispositif de reconnaissance (51-62, 64-67).

4. Appareil selon la revendication 3, dans lequel le repère (60) peut être reconnu par un contact électrique (61, 62).

5. Appareil selon la revendication 4, dans lequel le repère est une résistance électrique (60), en particulier une bande conductrice annulaire entourant le pourtour d'une section du corps d'impact (9).

6. Appareil selon la revendication 3, dans lequel le repère (57, 66, 67) peut être reconnu par une lumière, y compris un rayonnement infrarouge.

7. Appareil selon la revendication 6, dans lequel le repère (57, 66, 67) est un code clair/sombre, en particulier un code-barres.

8. Appareil selon la revendication 6 ou 7, dans lequel le corps d'impact (9) et une partie du reste de l'appareil comportent un aimant (53) et un détecteur magnétique (51) complémentaire, ou des éléments à forme complémentaire, pour l'indication de l'orientation lors du montage du corps d'impact (9).

9. Appareil selon l'une des revendications 6 à 8, dans lequel un conducteur optique (58, 64, 65) est intégré dans l'appareil pour la reconnaissance du repère, et notamment entre un tuyau interne (6) destiné au guidage de la pièce de butée (13) et un manchon externe (1) de l'appareil.

10. Appareil selon la revendication 3, dans lequel le repère (51) peut être lu par des ondes de fréquence radio, et un transpondeur (51), et le dispositif de reconnaissance comporte une bobine de réception/émission (52).

11. Appareil selon la revendication 10, dans lequel la bobine de réception/émission (52) est disposée entre un tuyau interne (6) destiné au guidage de pièce de butée (13) et un manchon externe (1) de l'appareil.

12. Appareil selon la revendication 10, dans lequel une bobine de réception/émission du dispositif de reconnaissance est prévue dans un appareil de base alimentant un appareil portatif de l'appareil.

13. Appareil selon la revendication 1, dans lequel le corps d'impact (9) ne comporte pas de repère, et le dispositif de reconnaissance (63) peut reconnaître le corps d'impact à l'aide de propriétés électromagnétiques, en particulier à l'aide d'une mesure d'inductance magnétique.
